# EUROPEAN PATENT APPLICATION

(11) **EP 0 780 127 A1**
(43) Date of publication of application: **25.06.1997**
(21) Application number: 96308852.1
(22) Date of filing: 05.12.1996
(51) Int. Cl.: A61K 31/57, A61K 31/58, A61K 31/56, A61K 31/495, A61K 31/445, A61K 31/55

(54) **A nasal spray containing a steroid and a antihistamine**

(30) Priority: 19.12.1995 US 574791
(71) Applicant: THE PROCTER & GAMBLE COMPANY, Cincinnati, Ohio 45202 (US)
(72) Inventor: Cramer, Ronald Dean, Cincinnati, Ohio 45215 (US)
(74) Representative: Woof, Victoria

(57) **Abstract**

The present invention relates to novel nasal spray compositions comprising a safe and effective amount of a glucocorticosteroid and an antihistamine possessing leukotriene inhibiting properties.

## Description

### TECHNICAL FIELD

The present invention relates to novel nasal spray compositions comprising a safe and effective amount of a glucocorticosteroid and an antihistamine.

### BACKGROUND OF THE INVENTION

Allergic disorders remain a leading cause of both acute and chronic illnesses the world over. These illnesses are often times present in the form of acute or chronic rhinoconjunctivitis. The symptoms of allergic rhinoconjunctivitis are reddening of the eyes, ocular secretions, nasal congestion, ocular and palatial irritation, sneezing and hypersecretion. These symptoms occur following exposure to allergens. The most common allergens are grass and/or tree pollens, hence, allergic rhinoconjunctivitis is most common during the spring and summer months.

The symptoms of allergic rhinoconjunctivitis are believed to be due primarily to the stimulation of H-1 receptors by histamine, followed by reflexive activation of parasympathetic nerves causing increases in nasal secretion and obstruction. Histamine is initially released from the tissue mast cells upon sensitization of the mast cells. This sensitization results when airborne allergens combine with specific IgE antibodies attached to mast cell membranes.

Antihistamines and/or decongestants have traditionally been the drugs of choice in treating allergic rhinoconjunctivitis. Other forms of therapy include the use of cromolyn sodium, hypertonic salt solutions or immunotherapy.

In addition, Hagen et al., U.S. Patent 4,767,612, discloses nasal corticosteroid therapy as an effective means of treating allergic rhinoconjunctivitis; and is herein incorporated by reference in its entirety. Notwithstanding the many disclosures in the area of allergic rhinoconjunctivitis, there is still a need for additional formulations which provide improved symptomatic relief with increased user acceptance and compliance.

The present inventor has found that by combining a nasal corticosteroid with a leukotriene inhibiting antihistamine, improved intranasal compositions result, providing improved relief of symptoms generally associated with either seasonal or perennial allergic rhinoconjunctivitis.

It is, therefore, an object of the present invention to provide pharmaceutical compositions having improved effectiveness in the treatment of symptoms generally associated with either seasonal or perennial allergic rhinoconjunctivitis.

A further object of the present invention is to provide a safe and effective method for treating the symptoms of seasonal or perennial allergic rhinoconjunctivitis.

These objects and other objects will become more apparent from the detailed description that follows.

### SUMMARY OF THE INVENTION

The present invention relates to pharmaceutical compositions for nasal administration comprising:
a) a safe and effective amount of a glucocorticoid selected from the group consisting of beclomethasone, flunisolide, triamcinolone, fluticasone, mometasone, budesonide, pharmaceutically acceptable salts thereof and mixtures thereof;
b) a safe and effective amount of a leukotriene inhibiting antihistamine selected from the group consisting of cetirizine, loratadine, azelastine, pharmaceutically acceptable salts thereof, optically active racemates thereof and mixtures thereof; and
c.) an intranasal carrier.

The intranasal carrier of the present invention is preferably aqueous.

The present invention also relates to a method for the treatment of symptoms associated with seasonal or perennial allergic rhinoconjunctivitis comprising the administration of a safe and effective amount of the intranasal pharmaceutical compositions of the present invention. By "symptoms of seasonal or perennial allergic rhinoconjunctivitis" or "symptoms associated with seasonal or perennial allergic rhinoconjunctivitis," is meant ocular and palatial irritation, ocular secretions, reddening of the eyes, sneezing, mucoid hypersecretion, nasal congestion and itching.

By "safe and effective amount," as used herein, is an amount that is effective to mitigate and/or treat the symptoms for which the active ingredient is indicated in a human without undue adverse side effects commensurate with a reasonable risk/benefit ratio.

By "leukotriene inhibiting antihistamine,"as used herein, is meant an antihistamine effective in inhibiting or reducing *in vivo* the biosynthesis of and/or cellular release of leukotrienes or otherwise modulating mammalian leukotriene levels.

The pH of the compositions is preferably from about 4.5 to about 9, more preferably from about 6 to about 7.

All percentages and ratios herein are by weight unless otherwise specified. Additionally, all measurements are made at 25°C unless otherwise specified.

### DETAILED DESCRIPTION OF THE INVENTION

The compositions of the present invention contain the essential components as well as various optional components as indicated below.

More specifically, the compositions of the instant invention are for nasal administration and contain a therapeutically effective amount of the herein described pharmaceutical agents. They are preferably provided as isotonic aqueous solutions, suspensions or viscous compositions which may be buffered to a selected pH.

### Essential Ingredients

### Glucocorticoid Agents

Agents within this class have potent glucocorticoid activity and weak mineralocorticoid activity. Glucocorticoid agents most useful to the present invention include those selected from the group consisting of beclomethasone, flunisolide, triamcinolone, fluticasone, mometasone, budesonide, pharmaceutically acceptable salts thereof and mixtures thereof.

When used in the compositions of the present invention, the glucocorticoid component is preferably present at a concentration of from about 0.001% to about 0.2%, more preferably from about 0.01% to about 0.1%.

### Leukotriene Inhibiting, Antihistaminic Agents

Antihistamines useful to the present invention are histamine H-1 receptor antagonists which also reduce mammalian leukotriene levels. Such H-1 receptor antihistamines may be selected from among the following groups of antihistamines: piperazines, phenothiazines, piperidines.

Examples of useful leukotriene inhibiting antihistamines include cetirizine, loratadine, azelastine and the like, optically active racemates thereof, pharmaceutically acceptable salts thereof and mixtures thereof. When used in the compositions of the present invention, the antihistamine component is preferably present at a concentration of from about 0.01% to about 4.0%, more preferably from about 0.01% to about 1%.

### Pharmaceutically-Acceptable Aqueous Nasal Carrier.

One other essential component of the present invention is a pharmaceutically-acceptable intranasal carrier. Preferred for use herein are aqueous saline solution carriers. These solutions which generally contain sodium chloride as the salt are fully described in Remington's Pharmaceutical Sciences, 17th edition (1985) p. 835, which is herein incorporated by reference. The salt is present in the solution at a level of about 0.01% to about 2%, preferably from about 0.5% to about 1.0%.

The combination of any of the above described antihistamines and glucocorticoids can be conveniently administered nasally to warm-blooded animals to elicit the desired therapeutic response by formulating it into a nasal dosage form, together with a nontoxic pharmaceutically-acceptable nasal carrier. Suitable nontoxic pharmaceutically-acceptable nasal carriers are known to those skilled in the art and are also fully disclosed in Remington's Pharmaceutical Sciences, 17th edition, 1985. Obviously, the choice of suitable carrier forms will depend on the exact nature of the particular nasal dosage form required, e.g., whether the drug(s) is to be formulated into a nasal solution (for use as drops or as a spray), a nasal suspension, a nasal ointment, a nasal gel or another nasal form. Preferred nasal dosage forms are solutions, suspensions and gels, which normally contain sodium chloride in a major amount of water (preferably purified water) in addition to the antihistamine and glucocorticoid. Minor amounts of other ingredients such as pH adjusters (e.g., an acid such as HCl), emulsifiers or dispersing agents, buffering agents, preservatives, wetting agents and jelling agents (e.g., methylcellulose) may also be present. Most preferably, the nasal composition is isotonic, i.e., it has the same osmotic pressure as blood and lacrimal fluid.

Preferably the composition is applied to the nasal mucosa via topical application of a safe and effective amount of the composition to treat nasal symptoms. The amount of the antihistamine and glucocorticoid combination and frequency of topical application to the nasal mucosa may vary, depending upon personal or medical needs, but it is suggested, as an example, that topical application range from about once per day to about four times daily, preferably twice daily, most preferably once daily. As a practical matter the selected therapeutic compositions will normally be prepared in unit dosage forms or actuations to contain therapeutically effective amounts of the selected antihistamine and glucocorticoid combination. In specific instances fractions of these dosage units or multiple dosage units will be employed. Typically, dosage units may be prepared to deliver from about 0.5 mcg to about 100 mcg of the glucocorticoid agent and from about 5 mcg to about 1000 mcg of the antihistaminic agent per spray actuation (e.g., 50 mg to about 200 mg of the spray composition). A typical dose contains one to four sprays per nostril.

### Optional Ingredients

Optional ingredients useful in the present invention include decongestants. Decongestants useful to the present invention may be selected from among the class of sympathomimetic agents; examples of which include pseudoephedrine, desoxyephedrine, propylhexedrine, phenylpropanolamine, xylometazoline, phenylephrine, tetrahydrozoline, naphazoline, oxymetazoline, tramazoline and pharmaceutically acceptable salts thereof. Also useful as decongestants are the 5-(2-imidazolinylamino)benzimedazole compounds. Mixtures of these decongestants can also be used.

When used in the compositions of the present invention, the sympathomimetic agents may be incorporated at concentrations, preferably, of from about 0.01% to about 0.5%, more preferably from about 0.05% to about 0.1%.

The compositions of the present invention may also contain antiallergics. Suitable antiallergics include, but are not limited to, cromolyn, ketotifen, N-allyl-(dichloro-3, 4-benzyl)-2-methylamino-2-propanol-1, AP-582 (Pharmaprojects No. 3055-under investigation by Ariad Pharmaceuticals), Andolast, oxatamide and pharmaceutically-acceptable salts thereof. Mixtures of these antiallergics may also be used.

Similarly, mucolytics such as acetylcysteine and anticholinergics such as ipratropium bromide may also be used in the compositions of the present invention.

Also of optional use in the compositions of the present invention are nonopiate analgesics such as oxaprozin. The intranasal use of oxaprozin is described in Namiki et al., Studies on improvement of pharmaceutical preparations prescribed in hospitals. VI. oxaprozin nasal spray, Drug Design and Delivery 1988;2:pp. 311-321, herein incorporated by reference. Further examples of preferred nonopiate analgesics include, but are not limited to, acetaminophen, acetylsalicylic acid, ibuprofen, etodolac, fenbuprofen, fenoprofen, flurbiprofen, indomethacin, ketoprofen, naproxen, pharmaceutically-acceptable salts thereof, optically active racemates thereof and mixtures thereof. Still further examples of such drugs are disclosed in U.S. Patent No. 4,522,828, to Sunshine et al., issued June 11, 1985; this patent being incorporated herein by reference in its entirety.

Synthetic opiate analgesics such as butorphanol may also be incorporated into the compositions of the present invention. The intranasal use of butorphanol is described in Baumel, Migraine: A pharmacologic review with newer options and delivery modalities, Neurology 1994;44(supp):pp. s13-s17, herein incorporated by reference. Further examples of preferred synthetic opioid analgesics include alfentanil, buprenorphine, fentanyl, meperidine, methadone, nalbuphine, natrexone, propoxyphene, pentazocine, sufenanil, pharmaceutically-acceptable salts thereof and mixtures thereof.

Compounds commonly known as lipoxygenase inhibitors and receptor antagonists are also optionally useful in the compositions of the present invention. Suitable lipoxygenase inhibitors are described in U.S. Patent 4,873,259, to Summers et al., issued October 10 1989 and European Patent Application 318093, both of which are herein incorporated by reference. Lipoxygenase antagonists suitable for use in the present invention include Zafirlukast (Accolate, Zeneca).

Leukotriene receptor antagonists may also be incorporated into the compositions of the present invention. Suitable examples include, but are not limited to, experimental agents such as LY171883, Wy-45,911, LY163443, ONO-RS-411 and ONO-RS-347 and ICI 198,615. A more detailed discussion of leukotriene receptor antagonists is found in Fleisch, J. H., Development of Cysteinyl Leukotriene Receptor Antagonists, Vol. 12 Advances in Inflammation Research 173-189 (A. Lewis et al. ed. 1988), herein incorporated by reference in its entirety.

Various aromatic components (e.g., aldehydes and esters) may also be used. These aromatics include, for example, menthol, camphor, eucalyptol, benzaldehyde (cherry, almond); citral (lemon, lime); neral; decanal (orange, lemon); aldehyde C-8, aldehyde C-9 and aldehyde C-12 (citrus fruits); tolyl aldehyde (cherry, almond); 2,6-dimethyl-octanal (green fruit); and 2-dodecenal (citrus, mandarin). Additional aromatic components suitable for use in the present invention include those described in U.S. Patent 4,136,163 to Watson et al., U.S. Patent 4,459,425 to Amano et al., and U.S. Patent 4,230,688 to Rowsell et al.; all of which are herein incorporated by reference. Mixtures of these aromatics can also be used.

The desired isotonicity of the compositions of this invention may be accomplished using, for example, the sodium chloride already present, or other pharmaceutically-acceptable agents such as dextrose, boric acid, citric acid, sodium tartrate, sodium phosphate, potassium phosphate, propylene glycol or other inorganic or organic solutes or mixtures thereof. Sodium chloride is preferred particularly for buffers containing sodium ions. Further examples of sodium chloride equivalents are disclosed in Remington's Pharmaceutical Sciences pp. 1491-1497 (Alfonso Gennaro 18th ed. 1990).

Viscosity of the compositions may be maintained at the selected level using a pharmaceutically-acceptable thickening agent. Methyl cellulose is preferred because it is readily and economically available and is easy to work with. Other suitable thickening agents include, for example, xanthan gum, microcrystalline cellulose, carboxymethyl cellulose, hydroxypropyl cellulose, hydroxypropyl methyl cellulose, hydroxymethyl cellulose, hydroxyethyl cellulose, carboxyvinyl polymer, carbomer, and the like or pharmaceutical salts thereof. Mixtures of such thickening agents may also be used. The preferred concentration of the thickener will depend upon the agent selected. The important point is to use an amount which will achieve the selected viscosity. Viscous compositions are normally prepared from solutions by the addition of such thickening agents.

Preferred compositions within the scope of this invention will contain from about 0.01% to about 5% of a humectant to inhibit drying of the mucous membrane and to prevent irritation. Any of a variety of pharmaceutically-acceptable humectants can be employed including, for example sorbitol, propylene glycol, polyethylene glycol, glycerol or mixtures thereof. As with the thickeners, the concentration will vary with the selected agent, although the presence or absence of these agents, or their concentration is not an essential feature of the invention.

Enhanced absorption across the nasal membrane can be accomplished employing a therapeutically acceptable surfactant. Typical useful surfactants for these therapeutic compositions include polyoxyethylene derivatives of fatty acid partial esters of sorbitol anhydrides such as Polysorbate 80, Polyoxyl 40 Stearate, Polyoxylethylene 50 Stearate and Octoxynol, as well as Oxyethylated tertiary octyl phenol formaldehyde polymer (available from Sterling Organics as tyloxapol) or mixtures thereof. The usual concentration is from 0.5% to 10% based on the total weight.

A pharmaceutically-acceptable preservative is generally employed to increase the shelf life of the compositions of the present invention. Benzyl alcohol is suitable, although a variety of preservatives including, for example, parabens, phenylethyl alcohol, thimerosal, chlorobutanol, phenylmecuric acetate or benzalkonium chloride may also be employed. The most preferred preservative system for use herein comprises a combination of benzalkonium chloride, chlorhexidine gluconate and disodium EDTA. A suitable concentration of the preservative will be from 0.001% to 2% based on the total weight, although there may be appreciable variation depending upon the agent selected Mixtures of these preservatives may also be used.

Other Optional Components. A variety of additional ingredients may be added to the emulsion compositions of the present invention. These additional ingredients include various polymers for aiding the film-forming properties and substantivity of the formulation, antioxidants, and agents suitable for aesthetic purposes such as fragrances, pigments, and colorings.

The compositions can also contain low levels of insoluble ingredients added, for example for visual effect purposes, e.g. thermochromic liquid crystalline materials such as the microencapsulated cholesteryl esters and chiral nematic (nonsterol) based chemicals such as the (2-methylbutyl) phenyl 4-alkyl(oxy)benzoates available from Hallcrest, Glenview, Illinois 60025, U.S.A. Mixtures of these ingredients may also be used.

### EXAMPLES

The following examples further describe and demonstrate embodiments within the scope of the present invention. The examples are given solely for the purpose of illustration and are not to be construed as limitations of the present invention, as many variations thereof are possible without departing from the spirit and scope of the invention.

### Example I

The intranasally administered pharmaceutical composition of the present invention is prepared by combining the following components utilizing conventional mixing techniques similar to that described below.

| **Component** | **Wgt %** |
|---|---|
| beclomethasone diproprionate, monohydrate | 0.042 |
| loratadine | 0.200 |
| avicel RC - 591¹ | 1.200 |
| dextrose | 5.100 |
| polysorbate 80 | 0.025 |
| benzalkonium chloride | 0.040 |
| phenylethyl alcohol | 0.250 |
| distilled water | q.s. to vol. |

| | |
|---|---|
| ¹microcrystalline cellulose and sodium carboxymethyl cellulose, supplied by FMC corporation. | |

In an appropriately sized vessel, the dextrose, polysorbate 80 and benzalkonium chloride are added one at a time to water with mixing, allowing each to dissolve or completely disperse before adding the next. To this is added, with mixing, a premixed slurry of the avicel and water. Upon forming a uniform solution, the beclomethasone, loratadine and phenylethyl alcohol are added. After all the ingredients are added, purified water is used to bring the batch to the appropriate weight.

Administration of approximately 0.4 grams of the composition is used for topical nasal application to provide relief from allergy or allergy-like symptoms.

### Example II

The intranasally administered pharmaceutical composition of the present invention is prepared by combining the following components utilizing conventional mixing techniques similar to that described in Example I.

| **Component** | **Wgt %** |
|---|---|
| flunisolide | 0.025 |
| cetirizine | 0.200 |
| propylene glycol | 2.000 |
| polyethylene glycol | 1.000 |
| sodium chloride | 0.900 |
| ethylenediamine tetraacetic acid | 0.050 |
| benzalkonium chloride | 0.010 |
| distilled water | q.s. to vol. |

Administration of approximately 0.4 grams of the composition is used for topical nasal application to provide relief from allergy or allergy-like symptoms.

### Example III

The intranasally administered pharmaceutical composition of the present invention is prepared by combining the following components utilizing conventional mixing techniques similar to that described in Example I.

| **Component** | **Wgt %** |
|---|---|
| triamcinolone acetonide | 0.050 |
| azelastine HCl | 0.070 |
| polysorbate 80 | 0.050 |
| glycerin | 2.000 |
| hydroxypropyl methyl cellulose | 1.000 |
| sodium chloride | 0.900 |
| ethylenediamine tetraacetic acid | 0.050 |
| benzalkonium chloride | 0.020 |
| distilled water | q.s. to vol. |

Administration of approximately 0.4 grams of the composition is used for topical nasal application to provide relief from allergy or allergy-like symptoms. Additionally, substantially similar results are also obtained using, in whole or in part, equivalent amounts of other glucocorticoid agents such as fluticasone, mometasone, budesonide, pharmaceutically acceptable salts thereof and mixtures thereof. Furthermore, the above described compositions may also contain a decongestant such as pseudoephedrine, phenylpropanolamine, phenylephrine, tetrahydrozoline, naphazoline, oxymetazoline, tramazoline, 5-(2-imidazolinylamino)benzimedazoles, optically active racemates thereof, pharmaceutically acceptable salts thereof and mixtures thereof. Those skilled in the art will quickly realize other suitable ingredients, diluents and dosage forms (or readily ascertain such using routine experimentation) which may further be incorporated into the above compositions without departing from the scope and spirit of the present invention.

## Claims

1. A pharmaceutical composition comprising:
a) a safe and effective amount of a glucocorticoid selected from the group consisting of beclomethasone, flunisolide, triamcinolone, fluticasone, mometasone, budesonide, pharmaceutically acceptable salts thereof and mixtures thereof;
b) a safe and effective amount of a leukotriene inhibiting antihistamine selected from the group consisting of cetirizine, loratadine, azelastine, pharmaceutically acceptable salts thereof, optically active racemates thereof and mixtures thereof; and
c.) an intranasal carrier.

2. A composition according to Claim 1 in the form of an isotonic aqueous solution
3. A composition according to Claim 1 or 2 wherein the glucocorticoid is selected from the group consisting of beclomethasone, budesonide, fluticasone and mixtures thereof.
4. A pharmaceutical composition according to any of Claims 1-3, which further comprises a sympathomimetic amine selected from the group consisting of pseudoephedrine, desoxyephedrine, propylhexedrine, phenylpropanolamine, xylometazoline, phenylephrine, tetrahydrozoline, naphazoline, oxymetazoline, tramazoline, 5-(2-imidazolinylamino)benzimedazoles, pharmaceutically acceptable salts thereof, optically active racemates thereof and mixtures thereof.
5. A pharmaceutical composition according to any of Claims 1-4, which further comprises a non-steroidal anti inflammatory agent, or optically active racemates thereof and mixtures thereof.
6. A pharmaceutical composition according to any of Claims 1-5, which further comprises a lipoxygenase inhibitor or antagonist, a leukotriene receptor antagonist, a nonopiate analgesic, a mucolytic, an antiallergic, and pharmaceutically acceptable salts thereof and mixtures thereof.
